(19) Europäisches Patentamt
European Patent Office
Office européen des brevets

(11) EP 4 214 185 B1

(12) EUROPEAN PATENT SPECIFICATION

(45) Date of publication and mention of the grant of the patent:
**17.07.2024 Bulletin 2024/29**

(21) Application number: **21773076.1**

(22) Date of filing: **06.09.2021**

(51) International Patent Classification (IPC):
***C07C 45/38*** $^{(2006.01)}$ ***C07C 47/127*** $^{(2006.01)}$

(52) Cooperative Patent Classification (CPC):
(C-Sets available)
**C07C 45/38;** Y02P 20/582 (Cont.)

(86) International application number:
**PCT/EP2021/074457**

(87) International publication number:
**WO 2022/058188 (24.03.2022 Gazette 2022/12)**

# (54) PROCESS FOR THE PRODUCTION OF GLYOXAL

VERFAHREN ZUR HERSTELLUNG VON GLYOXAL

PROCÉDÉ DE PRODUCTION DE GLYOXAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.09.2020 EP 20196611**

(43) Date of publication of application:
**26.07.2023 Bulletin 2023/30**

(73) Proprietor: **BASF SE**
**67056 Ludwigshafen am Rhein (DE)**

(72) Inventors:
- **ZAKZESKI, Joseph John**
  **67056 Ludwigshafen (DE)**
- **SIEMER, Michael**
  **67056 Ludwigshafen (DE)**
- **WALSDORFF, Christian**
  **67056 Ludwigshafen (DE)**
- **LUENSE, Jens**
  **67056 Ludwigshafen (DE)**
- **MOHL, Klaus-Dieter**
  **67056 Ludwigshafen (DE)**
- **HORN, Christian**
  **67056 Ludwigshafen (DE)**
- **STEFAN, Rittinger**
  **68199 Mannheim (DE)**
- **HAMMEN, Oliver**
  **67056 Ludwigshafen (DE)**
- **KARPOV, Andrey**
  **67056 Ludwigshafen (DE)**

(74) Representative: **BASF IP Association**
**BASF SE**
**GBI-C006**
**67056 Ludwigshafen (DE)**

(56) References cited:
**CN-A- 104 645 983**
**CN-A- 105 536 805**
**RU-C1- 2 599 247**
**US-A- 4 242 282**
**US-A- 4 511 739**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

(52) Cooperative Patent Classification (CPC): (Cont.)

C-Sets
**C07C 45/38, C07C 47/127**

## Description

The present invention relates to a continuous production of glyoxal in a reactor by oxidation of ethylene glycol in the gas phase with oxygen, cooling the reaction mixture to obtain an aqueous solution of glyoxal and a glyoxal depleted gas stream, and recycling the glyoxal depleted gas stream back to the glyoxal producing reactor as a diluting gas.

Glyoxal is an important intermediate and, due to the two directly adjacent C=O groups, mainly used as a synthesis building block in the production of higher refined chemicals or as a reducing agent. Typical synthesis building block applications are its use as a crosslinker of functionalized polymers (such as for cellulose, polyacrylamide, polyvinyl alcohol or keratin) and as a synthesis building block for the production of heterocycles.

Glyoxal is industrially usually produced by a heterogeneously catalyzed oxidation of ethylene glycol with oxygen in the gas-phase. The process goes back to so-called Laporte process which is described in GB 1,272,592. According to that vaporized ethylene glycol and an oxygen containing gas, which is preferably diluted with an inert diluent gas such as nitrogen to an oxygen concentration substantially lower than that of air, are processed at 180-600°C and atmospheric pressure over a copper or silver containing heterogeneous catalyst, which is promoted with phosphorus. The gaseous reaction mixture is then cooled to a temperature below the condensation point of ethylene glycol but above the condensation point of glyoxal to condense out ethylene glycol. The remaining gaseous mixture is then further cooled and quenched with water to obtain an aqueous solution of glyoxal. The remaining gas is withdrawn as off-gas.

It was already realized in the GB specification that by-products such as formaldehyde, formic acid, glycolic acid and glyoxylic acid are formed and that particularly a small quantity of formaldehyde is quenched into the aqueous glyoxal solution. Therefore, the GB specification proposes to purge the aqueous glyoxal solution at an elevated temperature of 90-105°C with steam or another inert gas to deplete the formaldehyde. As inert purge gas, part of the off-gas of the quench process can be used.

Later publications point out that there are various by-products formed during the oxidation of ethylene glycol and that the reaction mixture leaving the oxidation reactor contains beside glyoxal, water and carbon dioxide also some unreacted ethylene glycol and inter alia a variety of by-products such as carbon monoxide, formaldehyde, formic acid, glycol aldehyde, glycolic acid, glyoxylic acid, 2-hydroxymethyldioxolane, acetaldehyde and acetic acid.

In US 4,511,739, which relates to the preparation of glyoxal by oxidation of ethylene glycol in the presence of a silver containing catalyst, the formation of by-products was more deeply investigated. It was recognized that the amount of the by-products can be reduced if the hot reaction mixture leaving the oxidation reactor is quickly quenched with water or an aqueous glyoxal solution having a temperature of 0 to 130°C. To enable such quick cooling, it is proposed to provide a quench chamber directly below the exit of the oxidation reactor. At this proposal, unconverted ethylene glycol is also quenched into the aqueous glyoxal solution. As an alternative, also a heat exchanger which is directly located below the oxidation reactor is mentioned. This heat exchanger enables the quick cooling to condensate out ethylene glycol and to transfer the glyoxal containing gas phase to a water quench in which an aqueous solution of glyoxal is quenched out.

US 4,282,374 refers to the preparation of glyoxal by oxidation of ethylene glycol in the presence of a copper containing catalyst and points out that in the processes described in the prior art, the catalysts age relatively rapidly so that the glyoxal yield decreases soon. As a countermeasure, the US patent teaches to add a volatile phosphorus compound such as trimethyl or triethyl phosphate to the feed stream which then enables an extended catalyst life.

Since the processes described in GB 1,272,592, US 4,511,739 and US 4,282,374 are single-pass processes, it is required to add inert gas such as nitrogen to lower the oxygen concentration in the oxidation reactor in order to limit the reaction heat. Although such straight through pass processes are simple to perform, it is disadvantageous to additionally provide inert gases such as nitrogen as diluents in an amount which is a few dozen times higher than the amount of oxygen. Firstly, these additional inert gases have to be additionally prepared which causes additional work and energy demand, and, secondly, cause after the glyoxal was quenched out a huge amount of off-gas contaminated with organic by-products which has to be correctly disposed, e.g. via a flare.

RU 2,599,247 teaches a process for the preparation of glyoxal by (i) oxidizing ethylene glycol in the presence of a silver containing catalyst in an upper reaction zone of a vertical vessel, (ii) quickly quenching the reactor effluent with an aqueous solution of glyoxal in a quench chamber directly below the reaction zone, (iii) feeding the effluent of the quench chamber to a first absorption column and separating off therein an aqueous glyoxal product stream, whereby one part of the bottom stream is recycled to the upper zone of the first absorption column and the other part is recycled to the quench chamber, (iv) feeding the gaseous head stream of the first absorption column to a second absorption column which is interconnected with a third absorption column to absorb the remaining formaldehyde therein, (v) recycling back the remaining gaseous stream of the third absorption column to a vaporizer, whereby some purge is withdrawn and fresh oxygen containing gas added, and (vi) vaporizing fresh ethylene glycol into the gaseous and oxygen enriched recycle stream and feeding the combined stream as a mixed gaseous educt stream to the reaction zone. While the first absorption column is relevant for the separation and withdrawal of the aqueous glyoxal solution, the second and third absorption columns are intended to purify the recycle gas from formaldehyde. Formaldehyde is known as a major by-product. Its removal from the recycle gas avoids an accumulation in the process.

According to table 1 of the RU patent referring to examples 1 to 10, the recycling process in which the recycle gas was purified from formaldehyde by its absorption in water enable a glyoxal yield "Y" of 64.25 to 77.22 % at an ethylene glycol conversion "C" of 97.32 to 98.83 % from which glyoxal selectivity "S" can be calculated for each example by S = Y / C to be in the range of 65.0 to 79.2 %.

In contrast to single pass processes, the above-mentioned recycle process does not require the addition of external inert diluent gases since the recycle gas already functions as diluent and consequently also significantly reduces the amount of off-gas to be disposed. Thus, it considerably increases the efficiency of the whole process. However, although the amount of off-gas can be significantly reduced, a particular disadvantage of the described process is its relatively low glyoxal selectivity of below 80%, which means that more than 20% of the ethylene glycol is oxidized to unwanted by-products such as formaldehyde, formic acid, $C_2$ oxidation by-products as already mentioned above or even to carbon monoxide or carbon dioxide. These by-products may accumulate in a recycle process with time and negatively affect the behavior of the system as well as the purity of the water absorbed glyoxal if they are not separated off in an amount as they are formed.

CN 104645983 also deals with the purification of the recycle gas from organic by-products such as formaldehyde formed in the silver catalyzed oxidation of ethylene glycol to glyoxal. It is explained that the effect of a classical separation of the organic by-products by washing them out in a scrubber or an absorption column is only limited since only a part of the organic by-products is removed and the obtained loaded aqueous absorption liquid is to be disposed in an environment-friendly manner. To avoid the formation of such contaminated absorption liquids, the CN application teaches to oxidize the organic compounds in the recycle gas in the presence of a specific highly active combustion catalyst containing Cu, Ag, Mg, Pd or Pt on a La, Ce and Zr modified $Al_2O_3$ carrier. According to the CN application, such specific highly active combustion catalysts are, compared with common oxidation catalysts, sufficiently active to oxidize organic compounds in the presence of saturated water vapor at a low oxygen concentration. The CN application claims an ethylene glycol conversion of 99.1 to 100% but is silent on the glyoxal selectivity and glyoxal yield.

CN 105536805 picked up the idea of CN 104645983 oxidizing the organic compounds in the recycle gas of the oxidation of ethylene glycol to glyoxal and proposes to use a nano-copper cerium composite catalyst. These catalysts are claimed to have a higher activity than common composite oxides and completely oxidize the volatile organic compounds in the recycle gas, which are said to be in the range of several hundred ppm.

Whereas CN 104645983 and CN 105536805 focus on the removal of the organic by-products formed in the oxidation of ethylene glycol to glyoxal, other documents refer to the general impact of carbon monoxide on the behavior of different selective oxidation reaction systems other than the oxidation to glyoxal. One central issue here is the potential risk of forming a deflagration able or explosive mixture by the accumulation of carbon monoxide.

CN 110292929 relates to the selective oxidation of low-carbon alkanes such as propane to acrylic acid, isobutane to methacrylic acid or propane to propene in a recycle process, and describes that the recycle gas typically contains unreacted educt, organic by-products, water ($H_2O$), excess oxygen ($O_2$), carbon dioxide ($CO_2$) and carbon monoxide (CO). Whereas the unreacted educt is a valuable raw material for being recycled back, CO may accumulate in the recycle gas and may constitute a potential explosion source in the inlet of the recycle compressor. Since an oxidation of the recycle gas with common noble metal catalysts would also oxidize the unreacted educt, the CN application teaches to selectively oxidize CO in the presence of a CO selective Cu-based cheap metal oxide catalyst with a mill grade of 20-30 meshes, which relates to approximately 0.67 to 1.0 mm particle size. Due to the relatively high content of remaining unconverted organic educt in the recycling gas, a CO selective oxidation catalyst is described as beneficial.

US 10,029,974 relates to the selective oxidation of propane to acrylic acid in a recycle process and mentions that carbon monoxide would accumulate in the process with time forming an explosive mixture if it would not be removed. The proposed solution is the selective oxidation of carbon monoxide in the reactor effluent stream withdrawn before the acrylic acid is separated in the acrylic acid absorber.

WO 99/067,194 relates to the selective oxidation of n-butane to maleic anhydride in a recycle process and teaches to keep the concentration of carbon dioxide in the feed gas at $\leq$ 60 vol.-% and the molar ratio of carbon dioxide to carbon monoxide $\geq$ 1.5. Furthermore, it teaches that CO concentrations higher than the mentioned molar ratio to carbon dioxide present an inherent deflagration risk and that consequently higher carbon monoxide concentrations shall be reduced in the recycle gas, e.g. by selective oxidation or selective absorption and desorption. As a typical concentration of carbon monoxide in the feed gas, which is to be understood as deflagration resistant, a value of 24.0 vol.-% is mentioned in example A.

Notably WO 99/067,194 teaches that the limit concentration of carbon monoxide in a hydrocarbon and oxygen containing feed gas, which might cause a deflagration, is relatively high and significantly above 10 vol.-%, so that the person skilled in the art would not take care about carbon monoxide concentrations for deflagration safety issues in the lower percent region or below. This is consistent with the teaching of WO 2004/108,649 described in the following.

WO 2004/108,649 relates to the oxidation of alkanes and alkenes to carboxylic acids in a recycle process and teaches to intentionally maintain a concentration of carbon monoxide in the reactor feed gas stream of 1 to 20 vol.-%

since it was recognized that carbon monoxide within such range suppresses the formation of further carbon monoxide in the oxidation reaction and even increases the selectivity to the desired carboxylic acids.

Whereas the previously mentioned documents only teach that carbon monoxide concentrations in the reactor feed gases in the low two-digit percentage range are not susceptible to deflagration, WO 2004/108,649 teaches that a carbon monoxide concentration of 1 to 20 vol.-% might even suppresses the formation of further carbon monoxide in the oxidation reaction of alkanes and alkenes to carboxylic acids and even increases the selectivity to the desired oxidation product.

It was an object of the present invention to find an improved process for the continuous production of glyoxal by heterogeneously catalyzed oxidation of ethylene glycol in the gas phase, which is easy to perform, enables the use of common ethylene glycol oxidation catalysts and in which glyoxal can be obtained as an aqueous solution in high yield and high purity. Particularly, the new process shall enable a high selectivity in the conversion of ethylene glycol to glyoxal in order to better utilize the raw material and to reduce unwanted by-products which otherwise would have to be environment-friendly disposed. Furthermore, it shall be safe in its performance, constant in its high glyoxal quality and function stably over long operating times. Moreover, the process shall enable easy retrofitting of existing glyoxal production plants.

We have found a process for the continuous production of glyoxal by oxidation of ethylene glycol in the gas phase with oxygen, which comprises

(a) feeding an input stream (I) comprising 1 to 10 vol.-% ethylene glycol and 1 to 10 vol.-% oxygen based on the input stream (I) to a reactor (A),

(b) converting the input stream (I) in reactor (A) at a temperature of 180 to 700°C and a pressure of 0.05 to 1 MPa abs in the presence of a heterogeneous catalyst containing at least one of the elements Cu and Ag to an output stream (II) containing glyoxal, water, carbon dioxide and carbon monoxide,

(c) cooling the output stream (II) in a cooling zone (B) to obtain an aqueous solution of glyoxal (III) and a glyoxal depleted, carbon dioxide and carbon monoxide containing gas stream (IV),

(d) oxidizing carbon monoxide in the glyoxal depleted gas stream (IV) with oxygen in a reactor (C) in the presence of a heterogeneous oxidation catalyst to carbon dioxide, obtaining a carbon monoxide depleted gas stream (V),

(e) recycling the carbon monoxide depleted gas stream (V) as part of the input stream (I) to reactor (A), and

(f) adding ethylene glycol (VI) and oxygen (VII) as further part of the input stream (I),

in which the input stream (I) comprises 10 to 3000 vol.-ppm carbon monoxide based on the input stream (I).

The process of the invention follows the basic principles of the known recycle process for the production of glyoxal by oxidation of ethylene glycol in the gas phase with oxygen, at which an ethylene glycol and oxygen containing stream is oxidized in a reactor in the presence of a heterogeneous catalyst to a glyoxal containing stream, the glyoxal containing stream cooled to obtain an aqueous glyoxal solution which is separated off and a gaseous stream containing non-condensed gaseous compounds, and the gaseous stream recycled back to the reactor to dilute the ethylene glycol and oxygen containing inlet stream. In the course of finding an improved process which particularly enables a higher selectivity in the conversion of ethylene glycol to glyoxal, it was surprisingly found that the selectivity of the ethylene glycol conversion to glyoxal can be considerably increased at a consistently high ethylene glycol conversion if the carbon monoxide, which is produced as a by-product in the ethylene glycol oxidation and therefore present in the gaseous recycle stream, is significantly depleted in the gaseous recycle stream before being recycled back to the ethylene glycol oxidation reactor.

The process of the invention is described in the below paragraphs in more detail and illustrated in figure 1 by a simplified block diagram. The basic reaction equation is shown by equation (1).

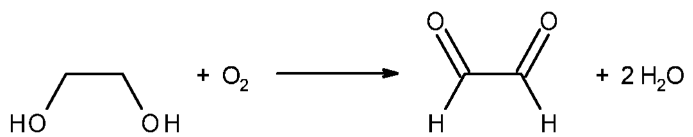

(1)

In the first step of the process according to the invention, which is named step (a), an input stream (I) comprising 1 to 10 vol.-% ethylene glycol and 1 to 10 vol.-% oxygen based on the input stream (I) is fed to a reactor (A). If the concentrations of ethylene glycol and oxygen would be lower than 1 vol.-%, the reactor feed would be too highly diluted

for an efficient production since the gas streams to be handled would become too large. On the other hand, concentrations of ethylene glycol and oxygen of above 10 vol.-% would cause too much heat by the exothermic reaction so that it would be more complex to control the reaction.

The concentration of ethylene glycol in the input stream (I) is preferably ≥ 1.5 vol.-%, more preferably ≥ 2 vol.-%, and preferably ≤ 5 vol.-% and more preferably ≤ 4 vol.-% based on the input stream (I) to a reactor (A). The concentration of oxygen in the input stream (I) is preferably ≥ 3 vol.-%, more preferably ≥ 4 vol.-%, and preferably ≤ 7 vol.-% and more preferably ≤ 6 vol.-% based on the input stream (I) to a reactor (A). The molar ratio between oxygen and ethylene glycol is generally 0.7 to 3, preferably ≥ 1.0, more preferably ≥ 1.2, and preferably ≤ 2.5 and more preferably ≤ 2.0. The concentration of oxygen is preferably chosen to provide sufficient oxygen content to allow sufficient oxidation of the ethylene glycol while being below the limiting oxygen concentration (LOC) to avoid risk of explosion. Inert gases such as nitrogen may serve to set the oxygen concentration and to prevent the formation of an explosive gas mixture; the same applies to argon, helium, carbon dioxide, or steam. Such diluents also serve to remove the exothermic heat of reaction. The addition of such gases is described further below.

Ethylene glycol is preferably added as stream (VI) as ethylene glycol of industrial grade having an ethylene glycol concentration of ≥ 98 wt.-%, more preferably ≥ 99 wt.-%, and typically some diethylene glycol, other glycols and water as main impurities. Some of ethylene glycol impurities may be removed, e.g. using an ion exchanger. Such ethylene glycol is typically obtained by hydrolysis of ethylene oxide.

Oxygen is preferably added as stream (VII) in the form of an oxygen containing gas having an oxygen concentration of ≥ 10 vol.-%, more preferably of ≥ 15 vol.-% and particularly preferably of ≥ 20 vol.-%. Although the preferred oxygen containing of the oxygen source is ≤ 50 vol.-% and more preferably ≤ 30 vol.-%, the addition of high concentrated oxygen of > 50 vol.-% and even pure oxygen is not excluded. However, the particularly preferred oxygen source is air and thus typically contains beside oxygen mainly nitrogen and some further compounds such as argon, carbon dioxide, neon, helium, carbon monoxide and water.

Beside ethylene glycol and oxygen, the input stream (I) typically contains inert gases such as nitrogen, argon and further noble gases, carbon dioxide, water vapor and the like, but also in a minor amount some organic compounds, hydrogen, and, as an essential feature of the invention, 10 to 3000 vol.-ppm carbon monoxide. Additionally, the input stream (I) may also contain auxiliary compounds for improving the performance of the catalyst.

Nitrogen, argon and further noble gases are mainly introduced as accompanying compounds of the air, if the oxygen is introduced by the addition of air, and then recycled back to stream (I) via streams (II), (IV) and (V). However, these gases may also be separately added, e.g. if the oxygen source does not contain sufficient inert gases. Generally, the input stream (I) contains 80 to 98 vol.-%, preferably 85 vol.-% to 95 vol.-% of these gases whereby nitrogen represents the major part of it.

Water vapor is mainly formed during the oxidation of ethylene glycol and some of the water, which is not condensed out and separated off together with the glyoxal is then also recycled back via streams (II), (IV) and (V) to stream (I). Furthermore, steam can also be separately added to stream (I). The concentration of water vapor in stream (I) is generally 1 to 20 vol.-%, preferably ≥ 3 vol.-% and preferably ≤ 15 vol.-%.

Carbon dioxide is mainly produced by over-oxidation of ethylene glycol along with formaldehyde, formic acid or even by its total oxidation. Since it is not or only to a very minor degree separated off together with the glyoxal, it is also recycled back as described above to stream (I). Its concentration in stream (I) is generally 1 to 10 vol.-%.

Organic compounds such as formaldehyde and formic acid are produced in the oxidation of ethylene glycol as by-products, and since they are only partly separated off together with the glyoxal, the remaining part remains in stream (IV) and is at least partly recycled back to stream (I). The nature of these organic compounds is described in more detail below in connection with step (b). Their total concentration in stream (I) is usually ≤ 200 vol.-ppm, preferably ≤ 100 vol.-ppm but typically ≥ 1 vol.-ppm, preferably ≥ 10 vol.-ppm and more preferably ≥ 20 vol.-ppm.

Since hydrogen is usually formed as a minor by-product in the catalytic conversion of ethylene glycol to glyoxal, and at least partly recycled back to stream (I), the input stream (I) also generally contains some minor amount of hydrogen in the order of ≤ 0.5 vol.-%, preferably ≤ 0.3 vol.-%, more preferably ≤ 0.2 vol.-% or even lower.

Auxiliary compounds for improving the performance of the catalyst, if desired, are mainly added to extend the service life and/or the activity of the catalyst. Such auxiliary compounds are typically phosphorus containing compounds such as trimethyl phosphate, triethyl phosphate, tri-isopropyl phosphate, tri-n-propyl phosphate, trimethyl phosphite, triethyl phosphite, triethyl phosphine oxide, diethyl methyl phosphonate, dimethyl methyl phosphonate, diethyl ethyl phosphonate or halogen containing compounds such as methyl chloride, methyl bromide, methyl iodide, ethyl chloride, ethyl bromide, ethyl iodide, dichloromethane, dibromomethane, diiodomethane, 1,2-dichloroethane, 1,2-dibromoethane, 1,2,-diiodoethane. Depending on the used catalyst, its status and the reaction conditions, the person skilled in the art generally knows which and how much of these auxiliary compounds are favorable. Their concentrations are generally very low and typically in the range of 1 to 100 wt.-ppm, preferably 1 to 25 wt.-ppm based on the weight of ethylene glycol.

Carbon monoxide is formed by over-oxidation of ethylene glycol similar to the formation of carbon dioxide but in which the oxidation stopped at the stage of the monoxide. Similar to carbon dioxide, it is not or only to a very minor

degree separated off together with the glyoxal and thus transferred to stream (IV). If the carbon monoxide is not specifically depleted in the recycle stream, which is the normal case in the prior art, it will be mainly recycled back and transferred to stream (I). Stream (I) would then typically contain around 0.5 to 2.0 vol.-% which relate to 5000 to 20000 vol.-ppm carbon monoxide and fed to reactor (A).

In the context of this invention and as already mentioned above, it was surprisingly found that the selectivity of the ethylene glycol conversion to glyoxal can be considerably increased if the carbon monoxide in the recycle stream is significantly depleted so that the reactor inlet stream (I) only contains a relatively low concentration of carbon monoxide of 10 to 3000 vol.-ppm. The difference in the carbon monoxide concentration between its typical value in the processes described in the prior art and the significantly reduced value according to this invention yields in a considerable increase of the glyoxal selectivity of several percent (relative and absolute) at a consistently high ethylene glycol conversion. For carbon monoxide concentrations between 2000 and 3000 vol.-ppm, the beneficial effect diminishes more and more, and above 3000 vol.-ppm, it is only very poor and almost disappears with a further increasing carbon monoxide concentrations. Regarding the lower limit, a permanent carbon monoxide concentration of < 10 vol.-ppm is very hard to maintain. The input stream (I) preferably comprises ≥ 50 vol.-ppm, more preferably ≥ 100 vol.-ppm, particularly preferably ≥ 300 vol.-ppm, and preferably ≤ 2500 vol.-ppm, more preferably ≤ 2000 vol.-ppm, particularly preferably ≤ 1500 vol.-ppm, very particularly preferably ≤ 1000 vol.-ppm, and most preferably ≤ 800 vol.-ppm based on the input stream (I).

The significant influence of the carbon monoxide concentration in stream (I) on the glyoxal selectivity and at the end also on the glyoxal yield was neither recognized nor discussed in the pertinent prior art found by the inventors. RU 2,599,247 teaches to selectively remove the by-product formaldehyde by absorbing it in water before the recycle stream is recycled back to the glyoxal reactor. CN 104645983 and CN 105536805 focus on the selective removal of the organic by-products such as formaldehyde but also failed to find out that carbon monoxide is present and that it has a significant detrimental effect on the glyoxal selectivity. All these three documents only focus on the removal of the organic by-products and are silent on carbon monoxide. Even though CN 110292929, US 10,029,974 and WO 99/067,194 describe that the formation of carbon monoxide in the selective oxidation of some low-carbon alkanes and its accumulation in the recycle stream may constitute a potential explosion source and teaches to oxidize the carbon monoxide in the recycle stream, it is not transferable to the selective oxidation of ethylene glycol to glyoxal since the risk of explosion due to the formation carbon monoxide is no topic for the glyoxal process, when it is operated below the limiting oxygen concentration (LOC) of glyoxal which is close to the LOC of carbon monoxide, and therefore, the person skilled in the art would not have been induced to transfer the teaching of CN 110292929 to the glyoxal process.

Since input stream (I) is generally provided to reactor (A) as a gaseous stream, but the ethylene glycol stream (VI) typically added as a liquid stream, it is advantageous to support its vaporization. Since the person skilled in the art is familiar with the vaporization of ethylene glycol in the glyoxal recycle process, it is only mentioned that vaporizers or spray towers are usually used, in which the liquid ethylene glycol is evaporated into the recycle stream (V), the oxygen containing stream (VII) or both. The evaporation energy is advantageously delivered by heat integration, mainly using heat produced by the oxidation in reactor (A). The liquid ethylene glycol stream (VI) as well as the gaseous streams (V) and (VII), respectively, may be preheated. For the sake of completeness, it is mentioned that also further compounds such as auxiliary compounds like trimethyl or triethyl phosphate as well as further inert gases like nitrogen can also be advantageously added to the vaporization supporting apparatus.

To further improve the intermixture of stream (I), it may be advantageous to pass the stream through a static mixer before it enters reactor (A). The use of such a static mixture is particularly recommended, if a further stream is added after the vaporization supporting apparatus. This may, for example, be the case if the liquid ethylene glycol (VI) is only vaporized into the recycle stream (V) and the oxygen containing stream (VII), e.g. in form of air, is added thereafter.

The input stream (I) is then fed in step (b) to reactor (A) and converted at a temperature of 180 to 700°C and a pressure of 0.05 to 1 MPa abs in the presence of a heterogeneous catalyst containing at least one of the elements Cu and Ag to an output stream (II) containing glyoxal, water, carbon dioxide and carbon monoxide. Reactor (A) may embrace one or more reaction apparatuses, but preferably, reactor (A) embraces only one reaction apparatus. In principle, suitable reaction apparatuses to be used according to the invention include those which are suitable for carrying out exothermic oxidation reactions in the gas phase and which can be operated continuously. Such reaction apparatuses are particularly fixed bed reactors known from the prior art. Suitable examples are staged ovens, fixed bed tubular reactors, shell and tube reactors, and plate heat exchanger reactors. Generally, shell and tube reactors are preferred. Such shell and tube reactors preferably contain 50 to 40000 parallel tubes with inner diameters of preferably 20 to 90 mm, more preferably ≥ 40 mm and more preferably ≤ 70 mm and a length of 1 to 10 m, more preferably 1.5 to 5 m. They may contain one, two or more independent tempering zones, which are connected to independently circulating heat carriers such as molten salts. The person skilled in the art is familiar with such reactors and their use in the selective oxidation of ethylene glycol to glyoxal.

The heterogeneous catalysts to be used in the process of the invention for the selective oxidation of ethylene glycol to glyoxal are virtually any heterogeneous catalysts which contain at least one of the elements Cu and Ag. Cu and Ag containing catalysts are known in the prior art to be suitable for the selective oxidation of ethylene glycol to

glyoxal. Nevertheless, some general aspects of suitable heterogeneous catalysts are shortly described in the following.

Suitable heterogeneous catalysts typically contain 1 to 100 wt.-%, preferably ≥ 20 wt.-%, more preferably ≥ 40 wt.-%, particularly preferably ≥ 60 wt.-% and very particularly preferably ≥ 90 wt.-% Cu, Ag or a mixture thereof. They may either contain only one of both elements, meaning only Cu, or only Ag, or both together as a mixed compound, for example as an alloy or a mixture of Cu and Ag containing particles. Irrespective of the detailed composition of the heterogeneous catalysts, Cu and Ag are generally present in the metallic state, whereby they can be partially oxidized forming the oxides $Cu_2O$, CuO and AgzO.

In addition to Cu and Ag, the heterogeneous catalysts may be doped with promotors, wherein compounds of groups 3 to 10 and 12 to 15 of the Periodic Table of the Elements are mentioned as preferred and compounds of groups 12 to 15 as particularly preferred elements. Very particularly preferred is P as promotor. If promotors are applied, they are typically applied in an total amount of up to 1000 wt.-ppm, preferably of ≤ 500 wt.-ppm and preferably of ≥ 10 wt.-ppm.

The heterogeneous catalysts can be full body catalysts (sometimes also called as unsupported catalysts) consisting essentially only of active mass, or supported catalysts.

In case of supported catalysts, the active mass is deposited on a carrier material. Suitable carrier materials typically contain mechanically stable and chemically rather inert compounds such as oxides, for instance $Al_2O_3$, $SiO_2$, TiOz or $ZrO_2$, or other inert compounds such as SiC or $Si_3N_4$. Although supported catalysts may also be formed with Cu as active component, they are mostly formed with Ag or combinations of Ag and Cu. The total content of Ag and Cu generally amounts to 50 to 95 wt.-%, whereas also an amount of less than 50 wt.-% is possible. Preferably, the total amount of Ag and Cu is 50 to 90 wt.-%. There are different methods under which the catalytically active mass can be deposited. One possibility is the wet impregnation of corresponding salts, their drying and calcination. The high contents can be achieved by repeating the procedure for several times. At the end, the active mass is reduced to the elementary metals. Another possibility is, for example, the electrolytical deposition of the active mass. The supported catalysts are preferably applied as coarse powder or grain with a particle size in the order of approximately 0.1 to 2.5 mm.

In case of full body catalysts, the catalysts essentially consist of active mass. They are typically produced by forming or partitioning the respective bulk material, which may be pure Cu, pure Ag, a mixture (an alloy) of Cu and Ag, or any of the mentioned material doped with promotors. In full body catalysts, the total content of Ag and Cu is preferably 90 to 100 wt.-%, more preferably ≥ 95 wt.-%, particularly preferably ≥ 98 wt.-% and very particularly preferably ≥ 99 wt.-%. Full body catalysts are typically applied in various shapes, such as rings, spheres, grains, meshes, wires or even wads (e.g. as scrunched thin wires). However, the preferred shape are rings. The outer dimensions of such rings (diameter, height) are typically in the order of 2 to 10 mm, preferably ≥ 2,5 mm, more preferably ≥ 3 mm, and preferably ≤ 8 mm, more preferably ≥ 7 mm and particularly preferably ≥ 6 mm. Preferably, the height amounts to 80 to 120% of the outer diameter. Furthermore, the thickness of the outer wall of the rings is typically 0.2 to 0.8 mm, preferably ≥ 0.3 mm and preferably ≤ 0.7 mm, more preferably ≤ 0.6 mm and particularly preferably ≤ 0.5 mm. As preferred examples of typical full body catalysts, Cu-rings of the dimensions 4 × 4 × (0.3-0.5) mm and 5 × 5 × (0.3-0.5) mm specified as outer diameter × height × thickness are mentioned.

The heterogeneous catalyst is typically provided in reactor (A) in the form of a bed. The bed may consist of a single layer or of two or more layers. These layers may consist of pure catalyst or be diluted with an inert material such as alumina, steatite, silicium carbide and the like. In case of different layers, these layers may differ in the geometry of the heterogeneous catalysts, the active mass of the heterogeneous catalysts including different dopants or even different layers of Cu containing catalysts and Ag containing catalysts, or in the presence or non-presence of inert material as diluents. Furthermore, reactor (A) may also contain separate layers of inert material, for example before the inlet stream contacts the first layer of the heterogeneous catalyst, for instance to ensure a thoroughly mixed or pre-tempered stream, or for example between two layers of the heterogeneous catalyst, for instance to better control the temperature of the exothermic oxidation reaction.

In a preferred process, the heterogeneous catalyst used in reactor (A) in step (b) contains Cu. This includes the use of only one layer of a Cu containing heterogeneous catalyst as well as the use of two or more layers of catalyst at which at least one layer contains a Cu containing heterogeneous catalyst. Of course, even if only one layer is applied, it may, beside Cu, also contain Ag and/or any dopants. In case of two or more layers, one or more layers may also be free of Cu and, for example, based on Ag as catalytically active component. In this regard, a twolayer filling of reactor (A) is mentioned in which the layer at the inlet side contains Cu as the main active component and at the outlet side Ag as the main active component, and vice versa. However, particularly preferred is a filling of reactor (A), which contains two catalytically active layers of ring-shaped catalysts based on Cu, whereby the layer at the inlet side has smaller rings than the layer at the outlet side.

The selective oxidation of ethylene glycol is performed at a temperature of 180 to 700°C and a pressure of 0.05 to 1 MPa abs. It is preferably carried out at a temperature of ≥ 200°C, more preferably at ≥ 250°C and particularly preferably at ≥ 300°C, as well as preferably at ≤ 650°C and more preferably at ≤ 600°C. With regard to the pressure, it is preferably carried out at ≥ 0.08 MPa abs, more preferably at ≥ 0.09 MPa abs and particularly preferably at ≥ 0.095 MPa abs, and preferably of ≤ 0.7 MPa abs and more preferably of ≤ 0.5 MPa abs. The typical pressure drop across the

reactor bed is in the range of 5 to 100 kPa, preferably 10 to 50 kPa.

Regarding the further and more detailed process conditions, Cu-based and Ag-based systems deviate from each other. Whereas a Ag-based system, in which the heterogeneous catalyst(s) in reactor (A) show(s) a Cu/Ag mass-ratio of 0 to 0.25, is typically operated adiabatically at a temperature of 500 to 600°C and a residence time of < 0.1 seconds across a short catalyst layer thickness of typically 1 to 20 mm, a Cu-based system, in which the heterogeneous catalyst(s) in reactor (A) show(s) a Ag/Cu mass-ratio of 0 to 0.25, is typically operated isothermally at a temperature of 350 to 450°C and a residence time of 1 to 3 seconds across a catalyst layer thickness of typically 250 to 300 cm. Based on the preference of a Cu-based system, it is particularly preferred that reactor (A) is operated isothermally and the Ag/Cu mass-ratio of the total amount of the catalyst filling in reactor (A) is 0 to 0.25. Isothermally means that the reaction heat is at least mainly removed by cooling. Of course, also at an isothermal operation, hotspots within the catalyst layers occur and are completely normal. In case of using a shell and tube reactor, the hotspot temperature is ≤ 150°C, preferably ≤ 120°C, more preferably ≤ 100°C and particularly preferably ≤ 80°C above the mean temperature of the cooling medium, which is defined as the arithmetic mean value between the outlet temperature and the inlet temperature of the cooling medium of the respective zone.

The gaseous hourly space velocity (GHSV) of the input stream (I) depends on the nature of the catalyst, the concentration of ethylene glycol and oxygen, and the further reaction conditions, but it is usually in the range of 1000 to 5000 $m^3/(m^3 \times h)$.

The obtained output stream (II) contains beside the desired reaction product glyoxal, water, carbon dioxide and carbon monoxide also further compounds which have been fed to reactor (A) and which remained unchanged, for instance added inert gases such as nitrogen and noble gases, unreacted ethylene glycol, unreacted oxygen, and organic compounds which either already have been fed to reactor (A) and which remained unchanged or which have been formed as oxidation by-products. Typical oxidation by-products are the $C_2$-compounds glycol aldehyde, glycolic acid and glyoxylic acid, the $C_1$-compounds formaldehyde and formic acid and compounds formed from these by-products such as 1,3-dioxolane or 2-hydroxymethyl-1,3-dioxolane.

The output stream (II) is then cooled in step (c) in a cooling zone (B) to obtain an aqueous solution of glyoxal (III) and a glyoxal depleted, carbon dioxide and carbon monoxide containing gas stream (IV). The cooling can be performed in different ways such as by using a heat exchanger, by injecting an aqueous cooling liquid such as water, by injecting a gaseous cooling stream, by using a combination of such methods, or by applying any other measurements which cause the gaseous reaction mixture to transfer the gaseous glyoxal into an aqueous liquid.

However, it is advantageous to already quickly lower the temperature of the hot output stream (II), which is also called to "quench" the output stream (II), before it is transferred to the cooling zone (B), to minimize the formation of by-products by a further oxidation of the glyoxal. Therefore, it is preferred to already precool the output stream (II) at, or shortly after the outlet of the reaction zone. Such precooling is usually performed by injecting an aqueous cooling liquid or a gaseous cooling stream into the output stream (II). The injected cooling medium can, for example, be liquid water or a liquid aqueous glyoxal solution which is sprayed into the hot gaseous output stream (II), inert gas such as a stream of nitrogen, or part of the gaseous recycle stream (IV) or (V). Irrespective of the reaction temperature in reactor (A), the hot output stream (II) is quickly cooled to preferably achieve a temperature of < 360°C and more preferably of ≤ 345°C.

The precooled output stream (II) is then fed to the cooling zone (B), whereby it is possible and generally advantageous to firstly further cool it down in a heat exchanger in order to use part of its thermal energy in the thermal network of the plant for heating up another streams, and then to condense out the glyoxal together with water formed by the conversion in step (b), preferably supported by quenching it with a stream of added water, to obtain an aqueous solution of glyoxal (III). The condensing of glyoxal can, for example, be performed in an apparatus such as a column or a quench scrubber, preferably equipped with internals, in which preferably water is sprayed in and which is operated at temperature under which water is liquid. Typically, such an apparatus is operated at a pressure of ≤ 0.2 MPa abs, preferably ≤ 0.15 MPa abs and a temperature of ≤ 60°C, preferably ≤ 50°C. The aqueous solution of glyoxal (III) can be easily withdrawn from the bottom of the apparatus, and the not condensed glyoxal depleted, carbon dioxide and carbon monoxide containing gas stream (IV) can easily be withdrawn from an upper region of the apparatus. Regarding the washing out of the glyoxal, it is advantageous to recirculate a part of the withdrawn aqueous solution of glyoxal (III) back to an upper part of the apparatus so that it trickles down together with the injected water in order to ensure a steady and stable liquid stream trickling down over the internals.

The cooling in step (c) causes the condensation of water and the formation of an aqueous glyoxal solution (III). The obtained aqueous glyoxal solution (III) is separated off and discharged as desired product. It usually contains 30 to 50 wt.-% glyoxal, preferably ≥ 38 wt.-% and preferably ≤ 45 wt.-%. Depending on the desired glyoxal concentration of the aqueous glyoxal solution (III), water is added and its amount controlled such that an aqueous glyoxal solution (III) within the mentioned concentration range is obtained. Beside glyoxal, also some by-products such as residual, unreacted ethylene glycol, formaldehyde, formic acid as well as $C_2$-by-products and compounds formed from these by-products as mentioned above are solved in the aqueous solution. The total content of the solved by-products is normally in the

range of 1 to 10 wt.-%, preferably ≥ 2 wt.-% and preferably ≤ 8 wt.-%.

The discharged aqueous glyoxal solution (III) may then be stored, purified if desired, or further processed in any way as usual.

The compounds of the output stream (II) which are not condensed and not solved in the aqueous glyoxal solution (III) remain in the glyoxal depleted, carbon dioxide and carbon monoxide containing stream (IV). Beside carbon dioxide and carbon monoxide, stream (IV) mainly contains inert gases such as nitrogen and noble gases, water vapor and oxygen which was not converted. Its composition varies with the reaction and process conditions, but stream (IV) typically contains

- 3 to 10 vol.-% carbon dioxide, preferably ≥ 5 vol.-% and preferably ≤ 8 vol.-%,
- 0.5 to 2.5 vol.-% carbon monoxide, preferably ≥ 0.75 vol.-%, more preferably ≥ 1.0 vol.-%, and preferably ≤ 2.25 vol.-% and more preferably ≤ 2.0 vol.-%,
- 2 to 20 vol.-% water vapor, preferably ≥ 3 vol.-% and preferably ≤ 10 vol.-%,
- 0 to 0.1 vol.-% organic by-products, preferably > 0 vol.-% and preferably ≤ 0.05 vol.-%,
- 0.1 to 2.0 vol.-% oxygen, preferably ≥ 0.3 vol.-% and preferably ≤ 1.5 vol.-%, and
- 0 to 1 vol.-% hydrogen, preferably 0 to 0.5 vol.-% hydrogen,

whereby the difference to 100 vol.-% are inert gases.

The steps (a), (b) and (c) above can easily be performed by applying process features and using process designs which are known in the prior art and/or which the person skilled in the art can easily implement.

In order to enable the re-use of the inert compounds of stream (IV) as a diluting gas in the reaction in step (a) and simultaneously ensure a carbon monoxide concentration of only 10 to 3000 vol.-ppm in the input stream (I), the carbon monoxide in the glyoxal depleted gas stream (IV) is oxidized with oxygen in step (d) in a reactor (C) in the presence of a heterogeneous oxidation catalyst to carbon dioxide to obtain a carbon monoxide depleted gas stream (V). To overcome the pressure drop mainly caused by the piping and reactor (C), a gas compressor is advantageously placed in stream (IV) between the cooling zone (B) and reactor (C).

The required factor of such a depletion and the carbon monoxide concentration in the depleted gas stream (V), respectively, can easily be determined by the person skilled in the art based on the carbon monoxide concentration in stream (IV) and the intended amount of gas stream (V) to be recycled back in relation to the other streams forming the input stream (I). To give a rough indication, the carbon monoxide concentration is generally depleted in reactor (C) to a range from slightly above 10 vol.-ppm, preferably ≥ 11 vol.-ppm, to ≤ 4000 vol.-ppm and preferably ≤ 3000 vol.-ppm in the depleted gas stream (V).

Basically, reactor (C) may embrace one or more reaction apparatuses. In principle, suitable reaction apparatuses to be used according to the invention include those which are suitable for carrying out slightly exothermic oxidation reactions in the gas phase and which can be operated continuously. Such reaction apparatuses are particularly fixed bed reactors known from the prior art, preferably fixed bed tubular reactors. Although a combination of two or more of such reactors is possible, it is generally preferred to use only one reaction apparatus. The flow through the catalyst bed can be aligned either axially or radially.

The oxidation can be effected adiabatically, autothermally or isothermally, but due to the low amount of the generated heat and a simpler design and operation, the oxidation is preferably effected adiabatically.

In principle, suitable heterogeneous oxidation catalysts to be used according to the invention include any heterogeneous catalysts which are suitable for the oxidation of carbon monoxide having a concentration in the low percentage range with oxygen being also present in a concentration in the low percentage range. These criteria are typically fulfilled for so-called combustion catalysts. Preferably, the heterogeneous oxidation catalyst contains at least one of the elements Pt, Ru, Pd, Au, Cu, V and Mn, more preferably Pt, Ru, Pd and Au, particularly preferably Pt, Ru and Pd and very particularly preferably Pt.

Although a full body catalyst such as a Pt-catalyst can also be used, it is preferred to use a supported catalyst. Supported catalysts are catalysts whose basic structure is given by a support material and the active component(s) deposited on their surface, e.g. by wet impregnation, coating or electrolytical deposition. Suitable support materials are, for example, oxides of Al, Si, Ce, Zr, Ti and mixtures thereof, which may optionally be doped with other metals such as rare earth metals like Y, Nd, La and Pr, with alkali earth metals such as Sr and Ba, or with transition metals such as Cu and Mn. Further suitable support materials are molecular sieves such as MCM-41 or SBA-15. The suitable support materials are typically characterized by a high BET surface area, which is usually in the range of 5 to 300 $m^2/g$, preferably ≥ 10 $m^2/g$ and preferably ≤ 200 $m^2/g$. The amounts of catalytically active components deposited on their surface mainly depend on the nature of the catalytically active component. For noble metals like Pt, Ru, Pd and Au, a low amount in the range of 0.01 to 0.5 g per gram supported oxidation catalyst typically suffices, whereas for Cu, V or Mn as catalytically active components a higher amount of 0.1 to 0.5 g per gram supported oxidation catalyst is preferred.

Basically, the full body catalysts as well as the supported catalysts may be applied in any shape which allows

a more or less steady flow of the glyoxal depleted gas stream (IV) through the bed of the heterogeneous oxidation catalyst in reactor (C). Suitable shapes include tablets, spheres, extrudates, pellets, rings, grains, meshes, monoliths and the like.

However, it was realized according to the invention that particularly in the recycle loop the provision of a low pressure drop is generally a central issue since with increasing pressure drop a higher starting pressure would be required, which causes at the end a higher energy consumption of the system or even a greater pumping equipment. Furthermore, existing plants might be limited in their pumping equipment and energy consumption so that the enabling of a low pressure drop in the recycle loop is typically also a central issue in the retrofitting of existing plants.

It has been found according to the invention that heterogeneous oxidation catalysts which only cause a low pressure drop in reactor (C) are particularly beneficial for the process of the invention. Such low pressure drops are notably achieved by using a heterogeneous oxidation catalyst with a monolithic shape. Therefore, a process is preferred, in which the heterogeneous oxidation catalyst used in reactor (C) in step (d) has a monolithic shape, or in other words, in which the heterogeneous oxidation catalyst is present in the form of a monolith.

For the present purposes, a monolith is a one-piece, parallelepipedal block having a plurality of continuous channels which are arranged parallel to one another and have a narrow cross section in the range from about 0.5 to 4 mm.

The monoliths are preferably formed by a ceramic material as support material onto which a catalytically active layer has been applied, preferably by the washcoating process. The most usual material for monolithic structures is cordierite (a ceramic material comprising magnesium oxide, silicon oxide and aluminum oxide in a ratio of 2 : 5 : 2). Other materials of which commercially available monolithic structures are made are metals, mullite (mixed oxide of silicon oxide and aluminum oxide, ratio = 2 : 3) and silicon carbide. These materials have, like cordierite, a low specific BET surface area, e.g. typically 0.7 $m^2/g$ for cordierite.

The preferred monoliths are characterized by a high density of cells, relating to a high number of cells per area. Preferably, a monolithic heterogeneous oxidation catalyst with a cell density of 4 to 250 cells per $cm^2$, more preferably $\geq$ 8 cells per $cm^2$ and more preferably $\leq$ 60 cells per $cm^2$ is used in step (d). A high number of cells per area increases the geometric surface area, so that the catalyst can be used more efficiently. At the other side, monoliths with a high number of cells per area are a somewhat more difficult to produce and to coat and they show a higher pressure drop over the reactor than monoliths with a lower number of cells per area. However, the pressure drop still remains very low for monoliths having a high cell count compared to a reactor packed with random packing elements, generally a factor of 10 lower. This effect can be attributed to the straight channels in the monolith.

The production of monoliths and their coating with the catalytically active components is well known the state of the art. Nevertheless, some aspects are shortly summarized below.

To produce monolithic ceramic elements, it is, for example, possible to produce a mixture of talc, clay and an aluminum oxide supplying component and silicon oxide, mix the mixture to form a molding composition, shape the mixture, dry the raw product and heat it at a temperature of from 1200 to 1500°C to give a ceramic which comprises mainly cordierite and which has a low coefficient of thermal expansion. This ceramic mass can then be powdered and mixed together with inorganic and/or organic additives, solvent (e.g. water), peptizing agent (e.g. acids) for setting the pH, and a permanent binder (e.g. a colloidal solution or sol) to form an extrudable paste. The paste is then extruded. Parameters in the extrusion which are important in respect of the properties of the monolith products are not only the quality of the nozzle and the type and properties of the materials used for producing the formable mixture but also the additives added, the pH, the water content and the force used in extrusion. The additives employed in extrusion are, for example, celluloses, $CaCl_2$, ethylene glycols, diethylene glycols, alcohols, wax, paraffin, acids and heat-resistant inorganic fibers. Apart from water, it is also possible to use other solvents such as ketones, alcohols and ethers. The addition of additives can lead to improved properties of the monoliths, for example formation of microcracks, which improves the thermal shock resistance, improved porosity and better absorption capacity and increased mechanical strength or low thermal expansion.

The bare monolithic structure can then be coated with a precursor of the catalytically active components, for example by wet impregnation with noble metal salts as they are also used for the impregnation of other supported catalysts. The macroporous structure of the ceramic monoliths aids the anchoring of the washcoat layer. The way of coating with the washcoat can be carried out by different methods. In one method, the macroporous support is (partly) filled with the washcoat material having a large surface area. In another method, the washcoat can be deposited as a layer in the pores of the ceramic support. The filling of the pores leads to a very strong interaction between monolith and washcoat since the major part of the washcoat layer is actually fixed in the pores of the support and not only bound to the outer surface of the monolith channels. This manner of coating is carried out using a solution (or a sol) of the material to be deposited or using a solution comprising very small colloidal particles. The disadvantage of carrying out the coating by filling of the pores is that the amount of coating which can be deposited is limited since the pores become completely filled at some time and the washcoat becomes inaccessible. The coated monoliths are then dried and calcined to finish the catalyst preparation.

Particularly preferred in step (d) is a monolithic heterogeneous oxidation catalyst containing Pt in an amount

of 0.035 to 7 $g/dm^3$. Notably, this monolithic heterogeneous oxidation catalyst has a cell density of 4 to 250 cells per $cm^2$ and particularly of 8 to 60 cells per $cm^2$.

Monoliths offer favorable conditions for enabling low pressure drops at narrow reactor cross sections and high flow velocities. By using monoliths as heterogeneous oxidation catalysts in reactor (C) in step (d), high gaseous hourly space velocities (GHSV) of $\geq$ 10000 $h^{-1}$, preferably $\geq$ 25000 $h^{-1}$ and particularly preferably $\geq$ 50000 $h^{-1}$ and at low pressure drops of $\leq$ 50 kPa, preferably $\leq$ 20 kPa and particularly preferable $\leq$ 10 kPa are possible.

The oxidation of carbon monoxide of stream (IV) in reactor (C) is typically performed at a temperature of 50 to 500°C and a pressure of 0.1 to 1 MPa abs. It is preferably carried out at a temperature of $\geq$ 100°C, more preferably $\geq$ 200°C and preferably at $\leq$ 400°C, more preferably at $\leq$ 350°C. With regard to the pressure, it is preferably carried out at $\geq$ 0.11 MPa abs, more preferably at $\geq$ 0.15 MPa abs and preferably at $\leq$ 0.5 MPa abs, more preferably $\leq$ 0.25 MPa abs. The gaseous hourly space velocity (GHSV) depends on the nature of the catalyst, the concentration of carbon monoxide and oxygen, the desired carbon monoxide concentration at the outlet of reactor (C) and the further reaction conditions, but is usually in the range of 1000 to 200000, preferably 10000 to 100000 $m^3/(m^3 \times h)$.

Depending on the used catalyst, also a part of the organic by-products may be oxidized.

Since the general performance of combustion reactions in the gas phase in the presence of combustion catalysts is known by the person skilled in the art, also the above-mentioned oxidation of carbon monoxide in stream (IV) can easily be implemented by the skilled person.

The resulting carbon monoxide depleted gas stream (V) is then in step (e) recycled back as part of the input stream (I) to reactor (A). Its further processing to form the input stream (I) is already described above in connection with input stream (I). The same applies for the addition of ethylene glycol (VI) and oxygen (VII) as further part of the input stream (I).

The process of the invention is operated continuously, which means that ethylene glycol (VI) and oxygen (VII) are continuously fed to the process and aqueous glyoxal (III) continuously withdrawn, whereby carbon monoxide in gas stream (IV) is continuously depleted and the carbon monoxide depleted gas stream (V) continuously recycled back to dilute the input stream (I).

If so-called further compounds such as inert gases like nitrogen are added to stream (I), which would already be the case if the oxygen would be added in the form of air, these further compounds would accumulate in the continuous recycle process if they would not be purged. The same applies for gaseous by-products which are also not separated off via the aqueous glyoxal stream (III). Such typical gaseous by-products are mainly carbon dioxide but also organic compounds which are not or only slightly removed with the aqueous glyoxal stream (III). In order to avoid such an accumulation, it is generally necessary to remove part of the recycle gas as a so-called purge. Such a purge might be discharged from the recycle gas as one purge stream or as a combination of two or more purge streams which are withdrawn from the recycle from different places. However, there are generally three principal possibilities, namely the discharge of the purge stream from stream (IV) before the oxidation reactor (C), the discharge of the purge stream from stream (V) after the oxidation reactor (C), or a combination of both.

As already mentioned before, a compressor is advantageously placed in stream (IV) between the cooling zone (B) and reactor (C). Such a compressor can generally be placed before or after the discharge of the purge stream. In order to minimize the amount of gas to be compressed, with the effect of reducing the size of the compressor and lowering the energy consumed, it is usually advantageous to place the compressor after the discharge of the purge stream. The mentioned advantage increases with an increasing amount of purge gas. By using air as oxygen containing gas in step (a), it is particularly advantageous to discharge the purge stream before feeding the recycle stream to the compressor.

Figure 2 shows a simplified block diagram in which the purge stream relating to stream (IV) is illustrated by stream (Villa) and the purge stream relating to stream (V) by stream (VIIIb). Figure 2 is to be understood that either purge stream (Villa) or purge stream (VIIIb) or both may be present. In order to minimize the gaseous stream through the oxidation reactor (C), it is particularly preferred to purge stream (IV) via purge stream (Villa). However, this does not exclude that a smaller part of the total purge might additionally be purged from stream (V) via purge stream (VIIIb).

Depending on the composition of stream (IV), or to be more specific on the concentration of the unconverted oxygen in relation to carbon monoxide and to organic compounds which may also be oxidized in the oxidation reactor (C) under the present conditions, and depending on the activity of the heterogeneous oxidation catalyst, it may absolutely suffice to oxidize the carbon monoxide in the oxidation reactor (C) without adding further oxygen. However, if the oxidation of ethylene glycol in step (b) is performed with no or only a slight excess of oxygen, it is advantageous to add some further oxygen to stream (IV). It is therefore preferred to add oxygen between steps (c) and (d) to the gas stream (IV) in order to enable or at least support the oxidation of carbon monoxide. The oxygen can be added as pure oxygen as well as by an oxygen containing gas such as air or the like. If oxygen is added, the addition of air is preferred. The amount of oxygen to be added can easily be determined by the person skilled in the art by a simple testing at the plant at which the amount of added oxygen is slowly increased until the desired concentration of carbon monoxide in stream (V) or stream (I), respectively, is attained. Irrespective of that, it is mentioned that at least a slight excess of oxygen

based on the theoretical amount being required for the oxidation of the intended amount of carbon monoxide plus the organic compounds which may also be oxidized, is preferred. However, oxygen may also be added in a much higher amount so that a notable amount of the excess oxygen remains unconverted in the carbon monoxide depleted stream (V) and already constitutes a notable part of the oxygen of stream (I), so that the addition via stream (VII) can be reduced. In an extreme variant, the total amount of oxygen of stream (I) may be added to the gas stream (IV) before entering reactor (C) so that the addition oxygen stream (VII) is formally shifted to an addition to the carbon monoxide depleted stream (V).

Figure 3 shows a simplified block diagram which is based on figure 2 and which additionally shows the addition of oxygen as stream (IX), forming stream (IVa).

Since stream (IV) leaving the cooling zone (B) typically has a temperature of ≤ 50°C but the oxidation of carbon monoxide in oxidation reactor (C) typically requires a temperature of ≥ 50°C, stream (IV) typically has to be heated before it enters the oxidation reactor (C). Such heating may be performed by adding external heat, for example by using a heat exchanger operated with steam, or by utilizing heat which is generated in the continuous glyoxal production process. Due to the exothermicity of the ethylene glycol oxidation to glyoxal and the required cooling of the output stream (II) to obtain an aqueous solution of glyoxal in step (c), it is particularly advantageous to utilize part of the reaction heat generated in step (b) to heat up stream (IV) before being oxidized in step (d). Therefore, it is preferred to heat the gas stream (IV) between steps (c) and (d) with the reaction heat gained in step (b). Particularly, such a heat transfer is performed by a heat exchanger (D) which is connected with streams (II) and (IV). Such a heat exchanger can, for example, be of any type of a heat exchanger which is applicable for such use, considering the nature and amount of the streams as well as the energy to be exchanged. As a typical heat-exchanger for that application, plate heat exchangers are mentioned. The person skilled in the art can easily select and/or design an appropriate heat exchanger.

Figure 4 shows a simplified block diagram which is based on figure 3, which additionally shows a heat exchanger (D) in the line of stream (II) incorporated between reactor (A) and cooling zone (B) in which stream (IV) is heated.

As already mentioned before, it is advantageous to quickly lower the temperature of the hot output stream (II) to minimize the formation of by-products by a further oxidation of the glyoxal. In that regard, a precooling by injecting a part of the gaseous recycle stream (IV) as one possible precooling measure was already mentioned beside others. Since an injection of part of stream (IV) to stream (II) does not require the addition of a further, additional component, and, due to its gaseous state, is highly fluid and therefore easily mixes with the gaseous output stream (II), the use of stream (IV) as precooling medium is very advantageous. Therefore, it is preferred to inject a part of gas stream (IV) to the output stream (II) in order to precool the output stream (II) and thus to suppress the formation of further by-products. The preferred amount of the gas stream (IV) to be injected to stream (II) for its quick cooling down enables the cooling down to a temperature as already mentioned above under step (c).

Figure 5 shows a simplified block diagram which is based on figure 4, which additionally shows the injection of part of stream (IV) to stream (II).

In a preferred embodiment, which is based on figure 5 as a simplified block diagram with the specific feature that the purge is only withdrawn via purge stream (Villa), ethylene glycol (VI) and a small amount of triethyl phosphate dissolved in ethylene glycol as a catalyst improving compound are mixed together and fed together with a stream of nitrogen to a vaporizer through which the recycle stream (V) is passed. The resulting stream is then mixed with air as oxygen containing stream (VII) to form the input stream (I) and, after having passed a static mixture, fed to reactor (A). Reactor (A) is a fixed bed tubular reactor containing a heterogeneous copper catalyst. The ethylene glycol is oxidized in reactor (A) to glyoxal and some by-products forming the output stream (II). The output stream (II) is then precooled by injecting a small part of the gas stream (IV), further cooled by a heat exchanger (D) through which another part of the gas stream (IV) is passed, quenched with water and fed to a packed column denominated as cooling zone (B) in which an aqueous solution of glyoxal (III) is withdrawn as bottom stream. The gaseous stream (IV) leaves the column at the top. From that stream, purge gas is withdrawn as purge stream (Villa) in an amount that enables a stable operation of the continuous recycle process. The remaining stream (IV) is fed to the above-mentioned heat exchanger (D), whereby a small part of stream (IV) is redirected to precool output stream (II) as described above. Since the heat exchanger (D) is intended to adjust the input temperature for the oxidation reactor (C), a part of stream (IV) may bypass the heat exchanger (D) so that the input temperature for the oxidation reactor (C) can be adjusted by the ratio between the amount passing the heat exchanger (D) and the amount bypassing the heat exchanger (D). The carbon monoxide of the heated gas stream (IV) is then oxidized in the oxidation reactor (C) in the presence of an oxidation catalyst, whereas Pt-containing catalysts coated on a monolith substrate are preferred. Reactor (C) is preferably a fixed bed tubular reactor and preferably operated at a pressure of ≥ 0.11 to ≤ 0.5 mPa abs, a temperature of ≥ 100 to ≤ 400°C and a GHSV of 10000 to 100000 1/h. Reactor (C) produces a carbon monoxide depleted stream (V) which is recycled to vaporizer as already mentioned before. Reactor (C) is operated in a way that at the end an input stream (I) with the targeted carbon monoxide content is obtained.

The process of the invention enables the continuous production of glyoxal by heterogeneously catalyzed oxidation of ethylene glycol in the gas phase in which glyoxal can be obtained as an aqueous solution in high yield and

high purity. The process is easy to perform and enables the use of common ethylene glycol oxidation catalysts and of common combustion catalysts for the processing of the recycle gas. Due to the inventive processing of the recycle gas by significantly lowering its carbon monoxide content, the process enables a high selectivity in the conversion of ethylene glycol to glyoxal at a constant high ethylene glycol conversion of above 99% and therefore a better utilization of the ethylene glycol raw material. Hence, the formation of unwanted by-products is reduced. Furthermore, the process is safe in its performance, constant in its high glyoxal quality and functions stably over long operating times. Moreover, it enables an easy retrofitting of existing glyoxal production plants, so that such plants can easily be upgraded.

Examples

The effect of carbon monoxide on the performance of the oxidation of ethylene glycol to glyoxal was investigated in a laboratory plant in a single pass.

Laboratory plant

The laboratory plant comprised a stainless steel reactor with an inner diameter of 24 mm and a height of 590 mm enclosed in an electrically-heated salt bath. The reactor was fitted with a multielement temperature sensor of outer diameter of 3.17 mm with 16 measurement points. The reactor was filled with 200 g of 5 × 5 × 0.3 mm copper rings (diameter × height × hole), which gave a catalyst bed height of 380 mm or a catalyst volume of 169 mL. The measurement points were separated by a distance of 2 cm until a height of 24 cm, at which point the next temperature elements were positioned at 28, 32 and 36 cm.

Performance of the experiments

The experiments were performed at atmospheric pressure and a constant salt bath temperature of 360°C. An aqueous solution containing ethylene glycol ("EG") and triethyl phosphate ("TEP") at a TEP/EG ratio of 1 ppm was placed in a container and then pumped into an evaporator operated at 180°C. The evaporated water/ethylene glycol mixture was introduced to a static mixer to which air, nitrogen and, where applicable, carbon monoxide and carbon dioxide were metered with the aid of mass flow controllers calibrated to each specific gas. The mixed reactant gas was then supplied to the reactor in an amount causing a gaseous hourly space velocity of gas stream of 1615 $h^{-1}$.

The compositions of the gaseous inlet and outlet streams of the reactor were monitored by online gas chromatography.

The ethylene glycol conversion was calculated by the following equation

$$X_{EG} = \frac{c(EG\ in) - \left(c(EG\ out) * \frac{c(N_2\ in)}{c(N_2\ out)}\right)}{c(EG\ in)} * 100 \qquad (1)$$

wherein

| | |
|---|---|
| $X_{EG}$ | = ethylene glycol conversion [%] |
| c(EG in) | = inlet concentration of ethylene glycol [vol.-%] |
| c(EG out) | = outlet concentration of ethylene glycol [vol.-%] |
| $c(N_2$ in) | = inlet concentration of nitrogen [vol.-%] |
| $c(N_2$ out) | = outlet concentration of nitrogen [vol.-%] |

The glyoxal selectivity was calculated by the following equation

$$S_{Gly} = \frac{c(Gly\ out) * \frac{c(N_2\ in)}{c(N_2\ out)}}{c(EG\ in) * X_{EG}} * 100 * 100 \qquad (2)$$

wherein

| | |
|---|---|
| $S_{Gly}$ | = glyoxal selectivity [%] |
| c(Gly out) | = inlet concentration of ethylene glycol [vol.-%] |

$c(N_2\ in)$ = inlet concentration of nitrogen [vol.-%]
$c(N_2\ out)$ = outlet concentration of nitrogen [vol.-%]
$X_{EG}$ = ethylene glycol conversion [%]

Since the concentrations in formulas (1) and (2) are specified in vol.-% which is equal to mol-%, and the molar amount of the gaseous mixture increases during the reaction due to its stoichiometry, the relative concentrations of c(EG out) and c(Gly out) at the outlet have to be corrected by a factor which reflects such increase. Such correction is made by multiplying the relative concentrations at the outlet with the factor $c(N_2\ in)$ / $c(N_2\ out)$.

Examples 1a to 1l

At example 1a, the laboratory plant was started up and operated as described above under "performance of the experiments". The adjusted composition of the input stream regarding ethylene glycol, oxygen, nitrogen, water, carbon monoxide and carbon dioxide is shown in table 1 and the laboratory plant operated under these conditions for one day. The compositions of gaseous inlet and outlet streams of the reactor were monitored by online gas chromatography and the ethylene glycol conversion $X_{EG}$ as well as the glyoxal selectivity $S_{Gly}$ calculated therefrom. The values shown in table 1 are the averaged values over one day of stable operation.

Example 1b covers day 2 of the operation. Its averaged values are also shown in table 1. The same applies accordingly for examples 1c covering day 3 up to example 1l covering day 12. After day 5, the concentration of CO in the inlet stream was raised from 0 vol.-% to 0.9 vol.-% and after day 6 from 0.9 vol.-% to 1.1 vol.-%, which are both typical CO concentrations of reactor inlet streams of recycle processes according to the prior art, in which CO is not removed.

While the ethylene glycol conversion $X_{EG}$ was unaffected by the CO concentration in the inlet stream showing high values in the range of 99.1 to 99.7%, the glyoxal selectivity showed a strong dependency on the CO concentration. At a CO concentration of 0.9 vol.-%, which is a typical value for the inlet stream of a recycle process, the glyoxal selectivity significantly decreased from 83.2 to77.9% and furthermore at a CO concentration of 1.1 vol.-% to only 75.5%. Although the CO concentration remained constant at 1.1 vol.-% between example 1g and 1l, the glyoxal selectivity steadily decreased with time to only 71.1% at example 1l five days later. The steady decrease between example 1g and 1l indicates the noxiousness of a larger concentration of CO over a longer time interval and the ongoing catalyst deactivation that comes with it.

In contrast to that, a high glyoxal selectivity of 80.1 to 83.2% could be obtained with an inlet stream containing no carbon monoxide. During the five days on stream with a CO concentration of 0.0 vol.-% (examples 1a to 1e), the glyoxal selectivity remained constant with a slight tendency to increase, which might have been caused by a slight activation of the catalyst during the first few days.

Examples 2 to 5

Examples 2 to 5 are long term examples performed in the laboratory plant, in which the long term stability of the glyoxal production without CO in the feed (example 2), with 300 vol.-ppm CO in the feed (example 3), with 1000 vol.-ppm CO in the feed (example 4) and with 2000 vol.-ppm CO in the feed (example 5) was investigated over about 50 days per example. At all examples, the laboratory plant was started up and operated as described above under "performance of the experiments". The feed compositions of examples 2 to 5 are shown in table 2. The compositions of gaseous inlet and outlet streams of the reactor were monitored by online gas chromatography and the ethylene glycol conversion $X_{EG}$ as well as the glyoxal selectivity $S_{Gly}$ calculated therefrom. The oxygen inlet concentration was varied over time in the range of 4.3 - 4.7 vol.-% to maintain the ethylene glycol outlet concentration in the range of 0.03 - 0.05 vol.-% which corresponds to an ethylene glycol conversion in the range of 98.5 - 99.0 %. The glyoxal selectivity over time of examples 2 to 5 is shown in figure 6. The markers show the averaged values over one day of stable operation.

The experimental results clearly show that the presence of 300 vol.-ppm CO in the feed did not negatively impact the glyoxal selectivity, compared with 0 vol.-ppm CO in the feed. Actually, a small increase of the average glyoxal selectivity was observed over 50 days of time on stream, as can be seen in figure 6 by the filled triangle markers (▲) in comparison with the empty squares (D).The presence of 1000 vol.-ppm CO already resulted over time in only a slightly decreased average glyoxal selectivity, as can be seen in figure 6 by the filled circular markers (•). A slightly larger decline of the average glyoxal selectivity over 50 days on stream was already observed in the presence of 2000 vol.-ppm CO, as can be seen in figure 6 by the x markers (×). However, a glyoxal selectivity of well above 70% was attained, even after 50 days on stream.

Table 1 Examples 1a to 1l

| Example | Time on stream [d] | EG [vol.-%] | $O_2$ [vol.-%] | $N_2$ [vol.-%] | $H_2O$ [vol.-%] | CO [vol.-%] | $CO_2$ [vol.-%] | X(EG) (%) | S (GLY) (%) |
|---|---|---|---|---|---|---|---|---|---|
| 1a | 1 | 3.3 | 4.7 | 84,5 | 7.5 | 0.0 | 0.0 | 99,6 | 82,5 |
| 1b | 2 | 3.3 | 4.6 | 84,2 | 7.5 | 0.0 | 0.0 | 99,7 | 81,9 |
| 1c | 3 | 3.3 | 4.6 | 83,1 | 7.5 | 0.0 | 0.0 | 99,7 | 82,1 |
| 1d | 4 | 3.3 | 4.6 | 82,6 | 7.4 | 0.0 | 0.0 | 99,6 | 82,2 |
| 1e | 5 | 3.3 | 4.6 | 83,4 | 7.4 | 0.0 | 0.0 | 99,1 | 83,2 |
| 1f | 6 | 3.3 | 4.4 | 83,2 | 7.4 | 0.9 | 0.0 | 99,6 | 77,9 |
| 1g | 7 | 3.3 | 4.4 | 82,1 | 7.4 | 1.1 | 0.0 | 99,7 | 75,5 |
| 1h | 8 | 3.3 | 4.4 | 83,5 | 7.4 | 1.1 | 0.0 | 99,6 | 74,5 |
| 1i | 9 | 3.3 | 4.4 | 83,5 | 7.4 | 1.1 | 0.0 | 99,5 | 74,5 |
| 1j | 10 | 3.3 | 4.4 | 82,5 | 7.3 | 1.1 | 0.0 | 99,4 | 74,1 |
| 1k | 11 | 3.3 | 4.4 | 83,0 | 7.4 | 1.1 | 0.0 | 99,5 | 72,5 |
| 1l | 12 | 3.3 | 4.4 | 83,6 | 7.3 | 1.1 | 0.0 | 99,6 | 71,1 |

Table 2 Feed composition of examples 2 to 5

| Example | EG [vol.-%] | O2 [vol.-%] | $N_2$ [vol.-%] | $H_2O$ [vol.-%] | CO [vol.-ppm] | $CO_2$ [vol.-%] |
|---|---|---|---|---|---|---|
| 2 | 3.3 | 4.3-4.7 | balance | 7.0 | 0 | 0.0 |
| 3 | 3.3 | 4.3-4.7 | balance | 7.0 | 300 | 0.0 |
| 4 | 3.3 | 4.3-4.7 | balance | 7.0 | 1000 | 0.0 |
| 5 | 3.3 | 4.3-4.7 | balance | 7.0 | 2000 | 0.0 |

Table 3 Performance data of examples 2 to 5

| Example | CO [vol.-ppm] | $CO_2$ [vol.-%] | Time on stream [d] | Average X(EG) (%) | Average S(GLY) (%) |
|---|---|---|---|---|---|
| 2 | 0 | 0.0 | 2-50 | 98,9 | 78,1 |
| 3 | 300 | 0.0 | 2-50 | 98,7 | 79,4 |
| 4 | 1000 | 0.0 | 2-50 | 98,7 | 76,4 |
| 5 | 2000 | 0.0 | 2-50 | 98,8 | 74,6 |

**Claims**

**1.** A process for the continuous production of glyoxal by oxidation of ethylene glycol in the gas phase with oxygen, which comprises

(a) feeding an input stream (I) comprising 1 to 10 vol.-% ethylene glycol and 1 to 10 vol.-% oxygen based on the input stream (I) to a reactor (A),
(b) converting the input stream (I) in reactor (A) at a temperature of 180 to 700°C and a pressure of 0.05 to 1 MPa abs in the presence of a heterogeneous catalyst containing at least one of the elements Cu and Ag to an output stream (II) containing glyoxal, water, carbon dioxide and carbon monoxide,
(c) cooling the output stream (II) in a cooling zone (B) to obtain an aqueous solution of glyoxal (III) and a glyoxal depleted, carbon dioxide and carbon monoxide containing gas stream (IV),

(d) oxidizing carbon monoxide in the glyoxal depleted gas stream (IV) with oxygen in a reactor (C) in the presence of a heterogeneous oxidation catalyst to carbon dioxide, obtaining a carbon monoxide depleted gas stream (V),
(e) recycling the carbon monoxide depleted gas stream (V) as part of the input stream (I) to reactor (A), and
(f) adding ethylene glycol (VI) and oxygen (VII) as further part of the input stream (I),

wherein the input stream (I) comprises 10 to 3000 vol.-ppm carbon monoxide based on the input stream (I).

**2.** The process according to claim 1, wherein the input stream (I) comprises 10 to 2000 vol.-ppm carbon monoxide based on the input stream (I).

**3.** The process according to any of claims 1 to 2, wherein the heterogeneous catalyst used in reactor (A) in step (b) contains Cu.

**4.** The process according to any of claims 1 to 3, wherein reactor (A) is operated isothermally and the Ag/Cu mass-ratio of the total amount of the catalyst filling in reactor (A) is 0 to 0.25.

**5.** The process according to any of claims 1 to 4, wherein the oxidation of carbon monoxide in step (d) is effected adiabatically.

**6.** The process according to any of claims 1 to 5, wherein the heterogeneous oxidation catalyst used in reactor (C) in step (d) contains at least one of the elements Pt, Ru, Pd and Au.

**7.** The process according to any of claims 1 to 6, wherein the heterogeneous oxidation catalyst used in reactor (C) in step (d) is a supported catalyst.

**8.** The process according to claim 7, wherein the heterogeneous oxidation catalyst has a monolithic shape.

**9.** The process according to claim 8, wherein the monolithic heterogeneous oxidation catalyst has a cell density of 4 to 250 cells per $cm^2$.

**10.** The process according to any of claims 8 to 9, wherein the monolithic heterogeneous oxidation catalyst contains Pt in an amount of 0.035 to 7 $g/dm^3$.

**11.** The process according to any of claims 8 to 10, wherein the pressure drop in reactor (C) in step (d) is $\leq$ 20 kPa.

**12.** The process according to any of claims 1 to 11, wherein between steps (c) and (d) oxygen is added to the gas stream (IV).

**13.** The process according to any of claims 1 to 12, wherein between steps (c) and (d) the gas stream (IV) is heated with the reaction heat gained in step (b).

**14.** The process according to any of claim 13, wherein the heat transfer is performed by a heat exchanger (D) which is connected with streams (II) and (IV).

**15.** The process according to any of claims 1 to 14, wherein a part of gas stream (IV) is injected to the output stream (II).

**Patentansprüche**

**1.** Verfahren zur kontinuierlichen Herstellung von Glyoxal durch Oxidation von Ethylenglycol mit Sauerstoff in der Gasphase, das Folgendes umfasst:

(a) das Zuführen eines Zufuhrstroms (I), der 1 bis 10 Vol.-% Ethylenglycol und 1 bis 10 Vol.-% Sauerstoff umfasst, bezogen auf den Zufuhrstrom (I), zu einem Reaktor (A),
(b) das Umwandeln des Zufuhrstroms (I) im Reaktor (A) bei einer Temperatur von 180 bis 700 °C und einem Druck von 0,05 bis 1 MPa absolut in Gegenwart eines heterogenen Katalysators, der mindestens eines der Elemente Cu und Ag enthält, in einen Ausgangsstrom (II), der Glyoxal, Wasser, Kohlendioxid und Kohlenmonoxid enthält,

(c) das Abkühlen des Ausgangsstroms (II) in einer Kühlzone (B), wodurch eine wässrige Lösung von Glyoxal (III) und ein Glyoxal-abgereicherter, Kohlendioxid und Kohlenmonoxid enthaltender Gasstrom (IV) erhalten werden,
(d) das Oxidieren von Kohlenmonoxid in dem Glyoxalabgereicherten Gasstrom (IV) mit Sauerstoff in einem Reaktor (C) in Gegenwart eines heterogenen Oxidationskatalysators zu Kohlendioxid, wodurch ein Kohlenmonoxid-verarmter Gasstrom (V) erhalten wird,
(e) das Zurückführen des Kohlenmonoxid-verarmten Gasstroms (V) als Teil des Zufuhrstroms (I) zum Reaktor (A) und
(f) das Hinzufügen von Ethylenglycol (VI) und Sauerstoff (VII) als weiteren Teil des Zufuhrstroms (I),

wobei der Zufuhrstrom (I) 10 bis 3000 Vol.-ppm Kohlenmonoxid umfasst, bezogen auf den Zufuhrstrom (I).

2. Verfahren nach Anspruch 1, wobei der Zufuhrstrom (I) 10 bis 2000 Vol.-ppm Kohlenmonoxid umfasst, bezogen auf den Zufuhrstrom (I).

3. Verfahren nach einem der Ansprüche 1 bis 2, wobei der im Reaktor (A) in Schritt (b) verwendete heterogene Katalysator Cu enthält.

4. Verfahren nach einem der Ansprüche 1 bis 3, wobei Reaktor (A) isotherm betrieben wird und das Ag/Cu-Massenverhältnis der Gesamtmenge der Katalysatorfüllung im Reaktor (A) 0 bis 0,25 beträgt.

5. Verfahren nach einem der Ansprüche 1 bis 4, wobei die Oxidation von Kohlenmonoxid in Schritt (d) adiabatisch bewirkt wird.

6. Verfahren nach einem der Ansprüche 1 bis 5, wobei der im Reaktor (C) in Schritt (d) verwendete heterogene Oxidationskatalysator mindestens eines der Elemente Pt, Ru, Pd und Au enthält.

7. Verfahren nach einem der Ansprüche 1 bis 6, wobei der im Reaktor (C) in Schritt (d) verwendete heterogene Oxidationskatalysator ein Trägerkatalysator ist.

8. Verfahren nach Anspruch 7, wobei der heterogene Oxidationskatalysator eine monolithische Form aufweist.

9. Verfahren nach Anspruch 8, wobei der monolithische heterogene Oxidationskatalysator eine Zelldichte von 4 bis 250 Zellen pro $cm^2$ aufweist.

10. Verfahren nach einem der Ansprüche 8 bis 9, wobei der monolithische heterogene Oxidationskatalysator Pt in einer Menge von 0,035 bis 7 $g/dm^3$ enthält.

11. Verfahren nach einem der Ansprüche 8 bis 10, wobei der Druckabfall im Reaktor (C) in Schritt (d) $\leq$ 20 kPa beträgt.

12. Verfahren nach einem der Ansprüche 1 bis 11, wobei zwischen den Schritten (c) und (d) Sauerstoff zum Gasstrom (IV) hinzugefügt wird.

13. Verfahren nach einem der Ansprüche 1 bis 12, wobei der Gasstrom (IV) zwischen den Schritten (c) und (d) mit der in Schritt (b) erhaltenen Reaktionswärme erwärmt wird.

14. Verfahren nach Anspruch 13, wobei die Wärmeübertragung mit einem Wärmetauscher (D) erfolgt, der mit den Strömen (II) und (IV) verbunden ist.

15. Verfahren nach einem der Ansprüche 1 bis 14, wobei ein Teil des Gasstroms (IV) in den Ausgangsstrom (II) eingespritzt wird.

**Revendications**

1. Procédé pour la production continue de glyoxal par oxydation d’éthylène glycol dans la phase gazeuse avec de l’oxygène, qui comprend

(a) l'alimentation d'un flux d'entrée (I) comprenant 1 à 10 % en volume d'éthylène glycol et 1 à 10 % en volume d'oxygène basé sur le flux d'entrée (I) à un réacteur (A),
(b) la conversion du flux d'entrée (I) dans le réacteur (A) à une température de 180 à 700 °C et une pression de 0,05 à 1 MPa abs en la présence d'un catalyseur hétérogène contenant au moins un des éléments Cu et Ag en un flux de sortie (II) contenant du glyoxal, de l'eau, du dioxyde de carbone et du monoxyde de carbone,
(c) le refroidissement du flux de sortie (II) dans une zone de refroidissement (B) pour obtenir une solution aqueuse de glyoxal (III) et un flux de gaz (IV) contenant du dioxyde de carbone et du monoxyde de carbone, appauvri en glyoxal,
(d) l'oxydation du monoxyde de carbone dans le flux de gaz (IV) appauvri en glyoxalavec de l'oxygène dans un réacteur (C) en la présence d'un catalyseur d'oxydation hétérogène en dioxyde de carbone, obtenant un flux de gaz appauvri en monoxyde de carbone (V),
(e) le recyclage du flux de gaz appauvri en monoxyde de carbone (V) comme partie du flux d'entrée (I) vers le réacteur (A), et
(f) l'ajout d'éthylène glycol (VI) et d'oxygène (VII) comme partie supplémentaire du flux d'entrée (I),

dans lequel le flux d'entrée (I) comprend 10 à 3 000 ppm en volume de monoxyde de carbone basé sur le flux d'entrée (I).

**2.** Procédé selon la revendication 1, dans lequel le flux d'entrée (I) comprend 10 à 2 000 ppm en volume de monoxyde de carbone basé sur le flux d'entrée (I).

**3.** Procédé selon l'une quelconque des revendications 1 à 2, dans lequel le catalyseur hétérogène utilisé dans le réacteur (A) à l'étape (b) contient du Cu.

**4.** Procédé selon l'une quelconque des revendications 1 à 3, dans lequel le réacteur (A) est exploité de manière isotherme et le rapport en masse Ag/Cu de la quantité totale du catalyseur remplissant le réacteur (A) est de 0 à 0,25.

**5.** Procédé selon l'une quelconque des revendications 1 à 4, dans lequel l'oxydation du monoxyde de carbone à l'étape (d) est effectuée de manière adiabatique.

**6.** Procédé selon l'une quelconque des revendications 1 à 5, dans lequel le catalyseur d'oxydation hétérogène utilisé dans le réacteur (C) à l'étape (d) contient au moins un des éléments Pt, Ru, Pd et Au.

**7.** Procédé selon l'une quelconque des revendications 1 à 6, dans lequel le catalyseur d'oxydation hétérogène utilisé dans le réacteur (C) à l'étape (d) est un catalyseur supporté.

**8.** Procédé selon la revendication 7, dans lequel le catalyseur d'oxydation hétérogène a une forme monolithique.

**9.** Procédé selon la revendication 8, dans lequel le catalyseur d'oxydation hétérogène monolithique a une densité cellulaire de 4 à 250 cellules par $cm^2$.

**10.** Procédé selon l'une quelconque des revendications 8 à 9, dans lequel le catalyseur d'oxydation hétérogène monolithique contient du Pt en une quantité de 0,035 à 7 $g/dm^3$.

**11.** Procédé selon l'une quelconque des revendications 8 à 10, dans lequel la chute de pression dans le réacteur (C) à l'étape (d) est $\leq$ 20 kPa.

**12.** Procédé selon l'une quelconque des revendications 1 à 11, dans lequel entre les étapes (c) et (d) de l'oxygène est ajouté au flux de gaz (IV).

**13.** Procédé selon l'une quelconque des revendications 1 à 12, dans lequel entre les étapes (c) et (d) le flux de gaz (IV) est chauffé avec de la chaleur de réaction acquise à l'étape (b).

**14.** Procédé selon l'une quelconque parmi la revendication 13, dans lequel le transfert de chaleur est effectué par un échangeur de chaleur (D) qui est connecté aux flux (II) et (IV).

**15.** Procédé selon l'une quelconque des revendications 1 à 14, dans lequel une partie du flux de gaz (IV) est injectée au flux de sortie (II).

Fig. 1

Block diagram (inventive)

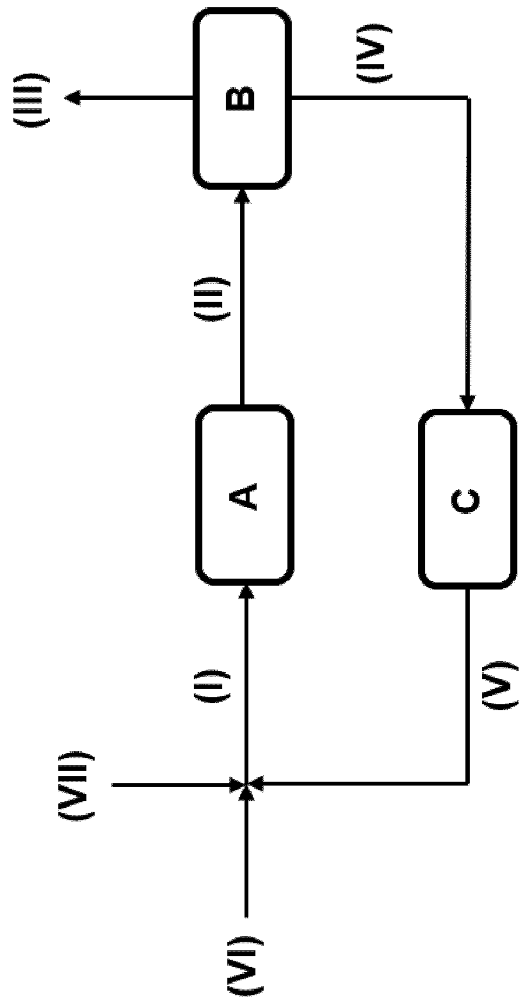

Fig. 2

Block diagram (inventive)

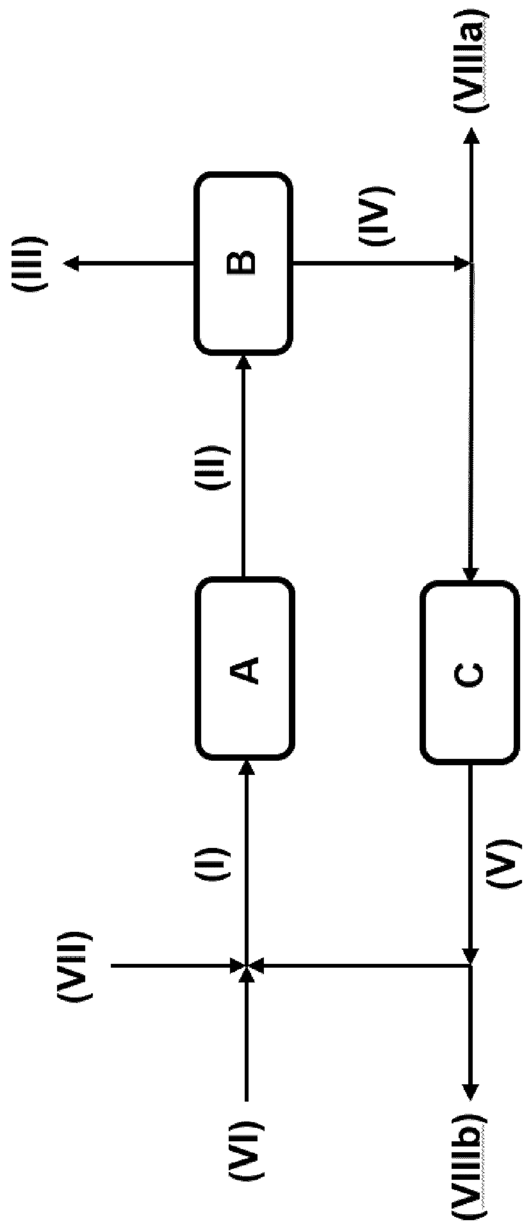

Fig. 3

Block diagram (inventive)

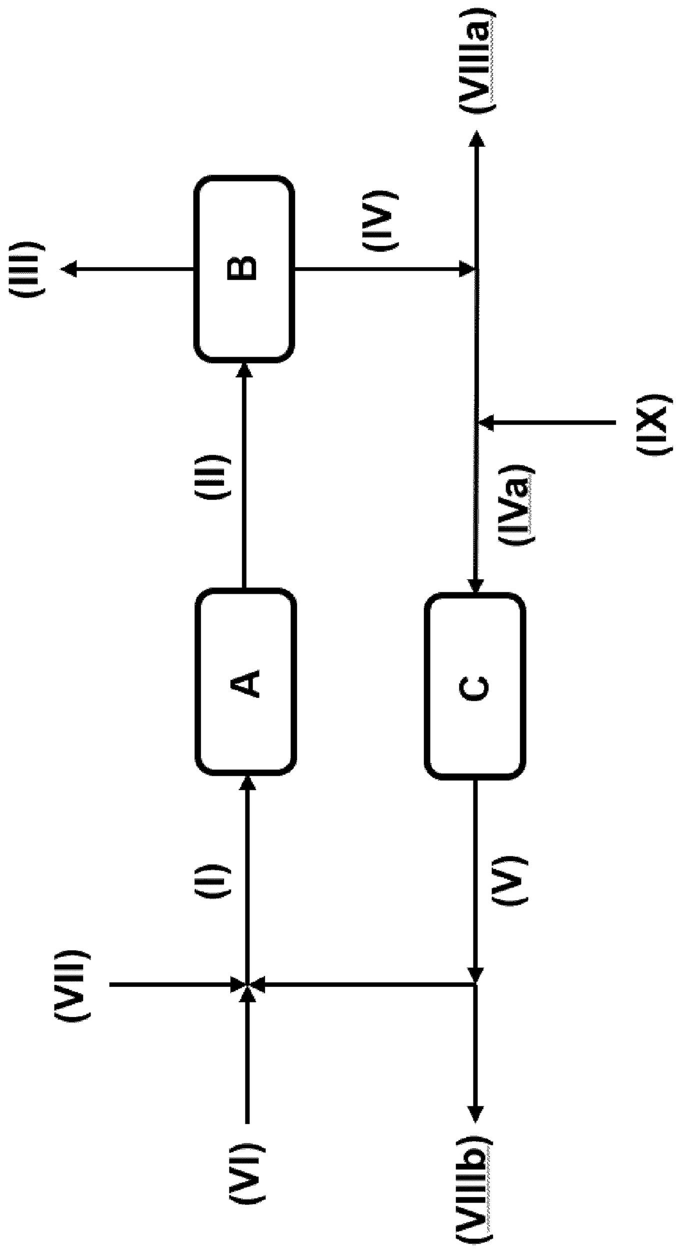

Fig. 4

Block diagram (inventive)

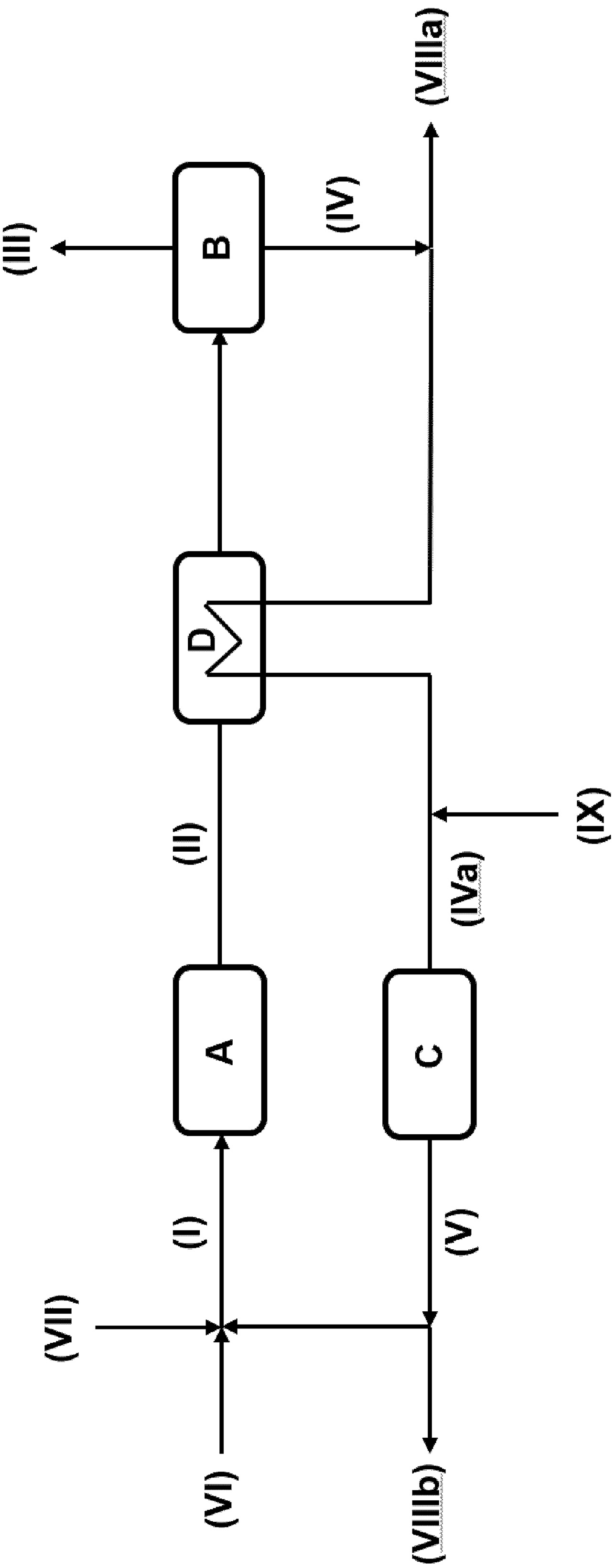

Fig. 5

Block diagram (inventive)

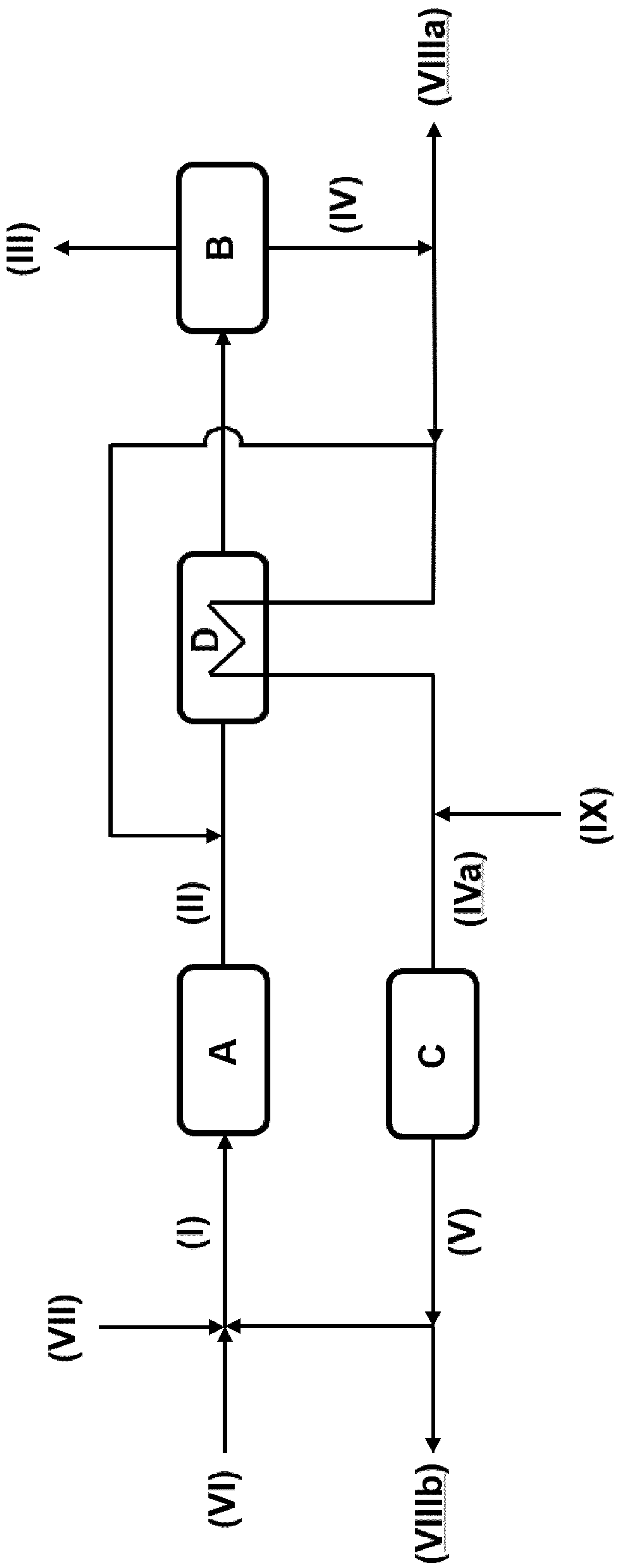

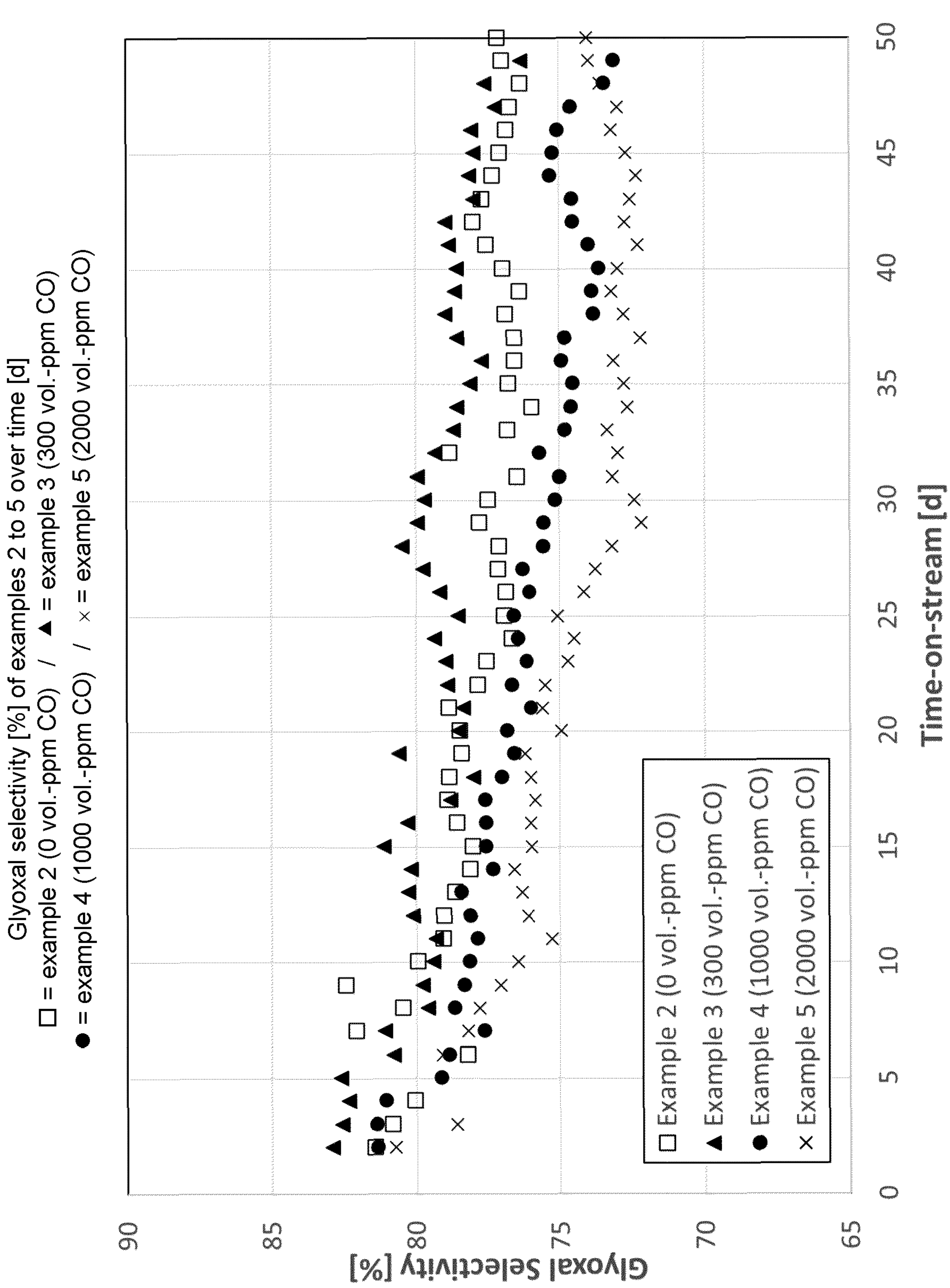

Glyoxal selectivity [%] of examples 2 to 5 over time [d]
□ = example 2 (0 vol.-ppm CO) / ▲ = example 3 (300 vol.-ppm CO)
● = example 4 (1000 vol.-ppm CO) / × = example 5 (2000 vol.-ppm CO)
Glyoxal Selectivity [%]
Time-on-stream [d]
□ Example 2 (0 vol.-ppm CO)
▲ Example 3 (300 vol.-ppm CO)
● Example 4 (1000 vol.-ppm CO)
× Example 5 (2000 vol.-ppm CO)
90
85
80
75
70
65
0
5
10
15
20
25
30
35
40
45
50


Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- GB 1272592 A
- US 4511739 A
- US 4282374 A
- RU 2599247
- CN 104645983
- CN 105536805
- CN 110292929
- US 10029974 B
- WO 99067194 A
- WO 2004108649 A